# FASCICULE DE BREVET EUROPEEN

(11) **EP 2 063 858 B1**
(45) Date de publication et mention de la délivrance du brevet: **01.08.2012**
(21) Numéro de dépôt: 07848362.5
(22) Date de dépôt: 21.09.2007
(51) Int. Cl.: A61K 8/73, A61K 8/97, A61Q 19/00

(54) **UTILISATION DE POLYSACCHARIDES DE HAUT POIDS MOLECULAIRE POUR UN EFFET TENSEUR IMMEDIAT**
VERWENDUNG VON POLYSACCHARIDEN MIT HOHEM MOLEKULARGEWICHT FÜR EINEN SCHNELLEN STRAFFUNGSEFFEKT
USE OF HIGH MOLECULAR POLYSACCHARIDES FOR THEIR IMMEDIATE STRENGTHENING EFFECT

(30) Priorité: 22.09.2006 FR 0653903; 08.03.2007 FR 0753714
(43) Date de publication de la demande: 03.06.2009
(73) Titulaire: Société Industrielle Limousine d'Application Biologique Dite SILAB, 19130 Objat (FR)
(72) Inventeur: PAUFIQUE, Jean, F-19130 Objat (FR)
(74) Mandataire: Cenatiempo, Julie Adeline Anne
(86) Numéro de dépôt international: PCT/FR2007/051992
(87) Numéro de publication internationale: WO 2008/035019

(56) Documents cités:
- EP-A2- 0 735 049
- WO-A-2005/048735
- FR-A- 2 874 930
- FR-A1- 2 838 343
- US-A1- 2003 147 830
- DATABASE WPI Week 200523 Derwent Publications Ltd., London, GB; AN 2005-220501 XP002432414 & RU 2 246 929 C2 (ARNEST STOCK CO) 27 février 2005 (2005-02-27)
- DATABASE WPI Week 199149 Derwent Publications Ltd., London, GB; AN 1991-356597 XP002432510 & JP 03 237102 A (KANEBO LTD) 23 octobre 1991 (1991-10-23)

## Description

La présente invention se rapporte à l'utilisation d'un principe actif riche en polysaccharides de haut poids moléculaire issu de fibres et/ou de son et/ou de graines d'avoine, présentant un effet anti-rides immédiat et/ou tenseur immédiat de la peau.

Pour paraître plus jeunes, beaucoup de gens souhaitent raffermir leur peau et atténuer les modifications physiques inesthétiques directement visibles liées au vieillissement cutané.

Le vieillissement de la peau résulte de diverses altérations provoquées par des facteurs à la fois génétiques et environnementaux. Il se manifeste notamment par la perte de la résistance mécanique et des propriétés viscoélastiques et liftantes du derme. La peau a alors tendance à s'étendre sous l'influence de son propre poids, provoquant ainsi des déformations de surface, la formation de rides et de plis disgracieux. L'épiderme perd également de son épaisseur et le microrelief cutané est modifié.

Pour lutter contre ce phénomène, on cherche donc des principes actifs cosmétiques permettant à la fois de lifter, de lisser le microrelief cutané, et d'améliorer les propriétés viscoélastiques de la peau.

Jusqu'à présent, pour répondre à leurs besoins liftants, les formulateurs avaient à leur disposition deux types de substances :
- des polymères synthétiques, collants, souvent difficiles à formuler car uniquement solubles dans l'alcool, et
- des protéines.

Aussi, la présente invention vise une autre voie moléculaire, pour pallier les inconvénients de l'art antérieur, en proposant un procédé d'obtention d'un principe actif à effet tenseur immédiat, efficace, soluble et stable dans l'eau, d'origine végétale et limitant au maximum la teneur en protéines.

A cet effet, l'invention a pour objet un procédé d'obtention d'un principe actif à effet anti-rides immédiat et/ou tenseur immédiat de la peau, caractérisé en ce qu'il consiste à extraire et à purifier des polysaccharides de haut poids moléculaire à partir de son et/ou de fibres et/ou de graines d'avoine, à solubiliser et à stabiliser ces polysaccharides dans l'eau.

Le principe actif selon l'invention peut être obtenu à partir de simples fibres d'avoine et/ou à partir de son d'avoine, résidu de la mouture d'avoine provenant du péricarpe des grains qui, en plus des fibres, contient des protéines, des sels minéraux et des vitamines, et/ou à partir de graines d'avoine.

Avantageusement, le principe actif obtenu, riche en polysaccharides de haut poids moléculaire, procure une sensation de peau tendue et tonique et présente un effet tenseur immédiat caractérisé par un lissage du microrelief cutané et une amélioration des propriétés mécaniques de la peau, ainsi qu'un effet anti-rides immédiat.

La présente invention est maintenant décrite en détail en s'appuyant sur des exemples non limitatifs de compositions, ainsi que sur des résultats d'essais regroupés en tableaux.

### I/ PROCEDE D'OBTENTION DU PRINCIPE ACTIF SELON L'INVENTION

Le procédé selon la présente invention comprend les étapes essentielles suivantes:
- une étape de solubilisation de son et/ou de fibres et/ou de graines d'avoine dans une solution basique, et
- une étape d'hydrolyse(s) enzymatique(s) successive(s) ou simultanée(s) des polysaccharides contenus dans le son et/ou fibres et/ou graines d'avoine, de façon à faciliter leur solubilisation sans rompre leur structure moléculaire, et une étape de déprotéinisation.

Selon un mode de réalisation de l'invention, pour faciliter la solubilisation des polysaccharides, on ajoute au moins un adjuvant de solubilisation dans la solution basique, préférentiellement un sel, un polyphosphate et/ou un oxydant.

La concentration en agent alcalin de la solution basique de solubilisation peut être ajustée pour que les propriétés physiques des polysaccharides ne soient pas altérées en sucres simples lors d'une hydrolyse.

Selon un mode de réalisation préféré, le procédé selon l'invention comprend la succession des étapes suivantes :
- solubilisation d'un son et/ou de fibres et/ou de graines d'avoine à raison de 30g/l à 300g/l, plus particulièrement de 50g/l à 150g/l, dans une solution basique,
- hydrolyse(s) enzymatique(s) successives ou simultanée(s) des polysaccharides,
- inactivation par traitement thermique ou chimique, pour bloquer les réactions enzymatiques,
- séparation des phases soluble et insoluble par filtration, décantation, et/ou centrifugation,
- concentrations successives,
- déprotéinisation par précipitation ou adsorption sélective,
- purification de la fraction active contenant les polysaccharides de haut poids moléculaire par ultrafiltration, et
- filtration stérilisante.

Avantageusement, le procédé selon l'invention permet la conservation des polysaccharides natifs issus de son et/ou fibres et/ou de graines d'avoine, tout en facilitant la faisabilité industrielle du principe actif.

### II/ CARACTERISATION DU PRINCIPE ACTIF OBTENU SELON L'INVENTION A PARTIR

### D'AVOINE

### II.1/ Matières sèches

On mesure le taux de matières sèches par passage à l'étuve à 105°C d'un échantillon de poids initial donné jusqu'à obtention d'un poids constant.

Le taux de matières sèches est compris entre 20 et 200 g/l, plus particulièrement entre 60 et 110 g/l.

### II.2/ Mesure du pH

Le pH mesuré par la méthode potentiométrique à température ambiante conduit à des valeurs comprises entre 4 et 8, plus particulièrement entre 5 et 6.

### II.3/ Détermination de la teneur en sucres totaux

On utilise la méthode de DUBOIS (DUBOIS M. & al [1956], Analytical chemistry, 28, n°3 p 350-356).

En présence d'acide sulfurique concentré et de phénol, les sucres réducteurs donnent un composé jaune-orangé.

A partir d'une gamme étalon, on peut déterminer le taux de sucres totaux d'un échantillon.

Le taux de sucres totaux du principe actif selon la présente invention est de 19 à 190 g/l, préférentiellement de 57 à 105 g/l.

Le ratio des sucres totaux sur le taux de matières sèches pour le principe actif selon la présente invention est supérieur à 50%, préférentiellement supérieur à 80%.

### II.4/ Degré de polymérisation moyen des polysaccharides

Le degré de polymérisation moyen des polysaccharides est déterminé par le ratio du taux de sucres totaux, sur le taux de sucres réducteurs.

Le dosage de sucres réducteurs est réalisé comme suit :
- on met le principe actif à doser en présence d'une solution de 4-hydroxybenzoïc hydrazide dans 0,5M de chlorure d'hydrogène et d'une solution de soude à 0,5M,
- on réalise une gamme étalon avec du glucose, et
- on mesure l'absorbance à 410nm pour déterminer la teneur en sucres réducteurs du principe actif par rapport à la gamme de glucose.

Le degré de polymérisation moyen des polysaccharides du principe actif selon la présente invention est supérieur à 40, préférentiellement supérieur à 60.

### II.5/ Taille des polysaccharides

La répartition de la taille des polysaccharides obtenus par la mise en oeuvre du procédé selon l'invention est réalisée par étude des chromatogrammes.

Les polysaccharides obtenus par la mise en oeuvre du procédé selon l'invention sont des polysaccharides de haut poids moléculaire. Ils présentent une taille des polysaccharides comprise entre 30 et 2 000 kDa. Préférentiellement 50% de la fraction polysaccharidique présente une taille comprise entre 70 et 700 kDa.

### III/ EVALUATION DE L'EFFET DU PRINCIPE ACTIF OBTENU SELON L'INVENTION A PARTIR D'AVOINE

### III-1/Evoluation de l'effet tenseur par Cutomètre

Cette étude a pour objectif d'évaluer l'effet tenseur d'un principe actif obtenu selon l'invention à partir de son d'avoine.

L'étude est réalisée sur volontaires à l'aide d'un Cutomètre.

Un Cutomètre est un dispositif muni d'une sonde que l'on applique sur la peau dans laquelle une dépression constante est maintenue. On mesure la profondeur de pénétration de la peau dans la sonde, sous l'effet de l'aspiration.

La peau, soumise à ces dépressions, se fatigue plus ou moins vite et les temps de réponse ainsi que les amplitudes mesurées permettent de déterminer des paramètres, notamment :
- une composante élastique, Ue, qui correspond à une déformation instantanée, et
- Uf, qui correspond à l'extensibilité.

Si Ue diminue, la peau est moins souple, donc plus tendue.

Si Uf diminue, la peau est moins extensible, donc plus tendue également. Le protocole opératoire est le suivant :
- on détermine une zone sur les avant-bras des volontaires, et on réalise une première série de mesures avec le Cutomètre,
- on applique le principe actif issu de son d'avoine obtenu selon l'invention à 4% en émulsion ou un placebo sur la zone déterminée, et
- deux heures après l'application, on effectue une nouvelle série de mesures sur la zone déterminée.

On utilise comme résultats de référence le SAB ( Sérum d'Albumine Bovine) dosé à 4%.

Les résultats obtenus pour le principe actif issu de son d'avoine selon l'invention sont exprimés par rapport au placebo dans le tableau suivant :

| | Efficacité cosmétique/placebo | |
|---|---|---|
| | (ΔUf) | (ΔUe) |
| SAB 4% | -5,0% | -7,1% |
| Principe actif selon l'invention | -8,4% | -9,9% |

On constate que le principe actif selon l'invention diminue la composante élastique et l'extensibilité de la peau : il présente un effet tenseur immédiat de la peau.

### III-2/Evaluation de l'effet tenseur en analyse sensorielle

L'objectif de cette étude est de quantifier in vivo l'efficacité tenseur d'un principe actif selon l'invention, obtenu à partir de son d'avoine, formulé à 10% en gel contre placebo.

Le test d'évaluation sensorielle consiste à faire évaluer en aveugle la sensation tenseur et non collante par un panel d'experts. L'étude est réalisée sur 15 volontaires sains au niveau de l'oeil et de la patte d'oie.

Le protocole opératoire est le suivant :
- à T moins 5 minutes, les volontaires démaquillent l'oeil et la patte d'oie sélectionnés,
- à T0, on applique 80µl d'un gel à tester : gel placebo, gel contenant 10% du principe actif selon l'invention issu de son d'avoine, gel contenant 5% de SAB, gel contenant 10% de SAB et gel contenant 20% de SAB, et
- à T 3 minutes, T 5 minutes et T 10 minutes, la sensation tenseur est évaluée sur une échelle de 0 à 10, à l'aide d'un curseur.

L'analyse des échelles est réalisée par la somme des scores aux trois temps.

Les différents gels sont testés aléatoirement sur plusieurs jours (un gel par jour).

Les résultats obtenus sont présentés dans le tableau ci-dessous :

| | Somme des scores des 3 temps |
|---|---|
| Placebo | 4,1 |
| Principe actif selon l'invention à 10% | 13,6 |
| SAB 5% | 8,2 |
| SAB 10% | 12,9 |
| % | 14,6 |

On constate qu'après une seule application, les experts reconnaissent le principe actif selon l'invention comme tenseur, non collant, et le score a une efficacité de 13,6, supérieure à celle du SAB dosée à 10%.

### III-3/Evaluation de l'effet anti-rides immédiat

Le but de cette étude est de quantifier l'efficacité anti-rides immédiat d'un principe actif selon l'invention, obtenu à partir de graines d'avoine, formulé à 4% en émulsion contre placebo.

L'étude est réalisée sur des volontaires sains de sexe féminin. L'efficacité anti-rides est mesurée par l'intermédiaire d'empreintes siliconées réalisées au niveau des pattes d'oie des volontaires.

L'analyse de ces empreintes à l'aide d'un profilomètre muni d'un analyseur d'images permet d'obtenir trois paramètres : le nombre de rides, la surface totale ridée et la longueur totale des rides.

L'étude est réalisée selon le protocole qui suit.

A T0, on détermine deux zones cutanées symétriques au niveau des pattes d'oie, une destinée à être traitée par placebo, l'autre par le principe actif, et on prend les empreintes de ces deux zones.

Après les prises d'empreintes, le placebo et le principe actif selon l'invention, issu de graines d'avoine, formulé à 4% sont appliqués sur les zones définies.

A T 2 heures, les empreintes sont prises sur les deux zones étudiées.

Les résultats obtenus pour le principe actif selon l'invention, issu de graines d'avoine, sont exprimés dans le tableau suivant en pourcentage par rapport à ceux obtenus pour le placebo :

| | Variation/placebo (%) |
|---|---|
| Nombre de rides | -11,5 |
| Surface totale ridée | -17,4 |
| Longueur totale | -13,9 |

On constate qu'après deux heures, en comparaison au placebo, le principe actif selon l'invention formulé à 4% diminue à la fois le nombre de rides, la surface totale ridée et la longueur totale des rides. Il présente donc un effet anti-rides immédiat.

### III-4/Evaluation de l'effet tenseur en analyse sensorielle

L'objectif de cette étude est de quantifier in vivo l'efficacité tenseur du principe actif selon l'invention, obtenu à partir de graines d'avoine, formulé à 4% en gel contre placebo.

Le test d'évaluation sensorielle consiste à faire évaluer en aveugle la sensation tenseur et non collante par un panel d'experts, formés à cette sensation tenseur. L'étude est réalisée sur 15 volontaires sains au niveau de l'oeil et de la patte d'oie.

Le protocole opératoire est le suivant :
- à T moins 5 minutes, les volontaires démaquillent l'oeil et la patte d'oie sélectionnés,
- à T0, on applique 80µl d'un gel à tester : un gel placebo ou un gel contenant 4% du principe actif selon l'invention issu de graines d'avoine, et
- à T 3 minutes, T 5 minutes et T 10 minutes, la sensation tenseur est évaluée sur une échelle de score allant de 0 à 10, à l'aide d'un curseur.

Les résultats obtenus, correspondant à la moyenne des scores aux trois temps, sont présentés dans le tableau ci-dessous :

| | Score moyen |
|---|---|
| Placebo | 2,1 |
| Principe actif selon l'invention à 4% | 4,4 |

On constate qu'après une seule application, les experts reconnaissent le principe actif selon l'invention comme tenseur, non collant et le score a une efficacité de 4,4.

### IV/ COMPOSITION COSMETIQUE INCLUANT LE PRINCIPE ACTIF SELON L'INVENTION :

La présente invention couvre aussi les compositions cosmétiques incluant le principe actif selon la présente invention dans différentes formes galéniques, notamment gel, solution, émulsion, crème....

Il convient alors d'analyser la stabilité des formes galéniques incluant le principe actif selon l'invention, ceci dans des proportions comprises entre 1 et 5 %.

La stabilité est caractérisée par une absence de précipitation de l'actif, une absence de crémage et une absence de déphasage.

On peut citer des formulations ayant montré une stabilité physique incluant 5% de principe actif selon l'invention.
- Gel limpide :: - Carbopol : 0,5% avec Triéthanolamine : qsp pH=6,5
- Conservateur : 0,7%
- Principe actif : 5,0%
- Eau : 93,8%
- Gel opaque :: - Sépigel 305 : 2,0%
- Conservateur : 0,7%
- Principe actif : 5,0%
- Eau : 92,3%
- Gel émulsionné :: - Montanov 202 : 3,0%
- Isopropyl palmitate : 12,0%
- Conservateur : 0,7%
- Viscolam AT 64 : 2,0%
- Principe actif : 5,0%
- Eau : 77,3%
- Emulsion non ionique :: - Montanov 202 : 3,0%
- Simulsol 165 : 2,0%
- Isopropyl palmitate : 20,0%
- Conservateur : 0,7%
- Principe actif : 5,0%
- Eau : 69,3%
- Emulsion anionique :: - Acide stéarique : 7,0%
- Triethanolamine : 3,5%
- Isopropyl palmitate : 20,0%
- Conservateur : 0,7%
- Principe actif 5,0%
- Eau : 63,8%
- Emulsion cationique :: - Quaternium-82 : 5,0%
- Alcool cétylique : 2,0%
- Alcool cétéarylique : 1%
- PEG100 stéarate : 1%
- Isopropyl palmitate : 15,0%
- Conservateur : 0,7%
- Principe actif : 5,0%
- Eau : 70,3%

De plus, des tests ont montré la compatibilité du principe actif avec les matières premières utilisées en cosmétique.

## Revendications

1. Utilisation pour présenter un effet tenseur cutané immédiat, de polysaccharides de haut poids moléculaire compris entre 30 et 2 000 kDa, obtenus à partir de son et/ou de fibres et/ou de graines d'avoine et par mise en oeuvre d'un procédé consistant à extraire et purifier des polysaccharides de haut poids moléculaire à partir de son et/ou de fibres et/ou de graines d'avoine, à solubiliser et à stabiliser ces polysaccharides dans l'eau, comprenant au moins les étapes suivantes :
- solubilisation de son et/ou de fibres et/ou de graines d'avoine dans une solution basique,
- hydrolyse(s) enzymatique(s) successive(s) ou simultanée(s) des polysaccharides contenus dans le son et/ou les fibres et/ou de graines d'avoine, et
- déprotéinisation, dans une composition cosmétique.

2. Utilisation, pour procurer une peau tendue et tonique, de polysaccharides de haut poids moléculaire compris entre 30 et 2 000 kDa, obtenus à partir de son et/ou de fibres et/ou de graines d'avoine et par mise en oeuvre d'un procédé consistant à extraire et purifier des polysaccharides de haut poids moléculaire à partir de son et/ou de fibres et/ou de graines d'avoine, à solubiliser et à stabiliser ces polysaccharides dans l'eau, comprenant au moins les étapes suivantes :
- solubilisation de son et/ou de fibres et/ou de graines d'avoine dans une solution basique,
- hydrolyse(s) enzymatique(s) successive(s) ou simultanée(s) des polysaccharides contenus dans le son et/ou les fibres et/ou de graines d'avoine, et
- déprotéinisation dans une composition cosmétique.

3. Utilisation, pour présenter un effet anti-rides immédiat, de polysaccharides de haut poids moléculaire compris entre 30 et 2 000 kDa, obtenus à partir de son et/ou de fibres et/ou de graines d'avoine et par mise en oeuvre d'un procédé consistant à extraire et purifier des polysaccharides de haut poids moléculaire à partir de son et/ou de fibres et/ou de graines d'avoine, à solubiliser et à stabiliser ces polysaccharides dans l'eau, comprenant au moins les étapes suivantes :
- solubilisation de son et/ou de fibres et/ou de graines d'avoine dans une solution basique,
- hydrolyse(s) enzymatique(s) successive(s) ou simultanée(s) des polysaccharides contenus dans le son et/ou les fibres et/ou de graines d'avoine, et
- déprotéinisation, dans une composition cosmétique.

4. Utilisation de polysaccharides de haut poids moléculaire selon l'une des précédentes revendications, **caractérisé en ce que** lesdits polysaccharides sont des polysaccharides obtenus par la mise en oeuvre d'un procédé comprenant la succession des étapes suivantes :
- solubilisation de son et/ou de fibres et/ou de graines d'avoine dans une solution basique, à raison de 30g/l à 300g/l,
- hydrolyse(s) enzymatique(s) successive(s) ou simultanée(s) des polysaccharides contenus dans le son et/ou les fibres et/ou de graines d'avoine,
- inactivation par traitement thermique ou chimique, pour bloquer les réactions enzymatiques,
- séparation des phases soluble et insoluble par filtration, décantation, et/ou centrifugation,
- concentration(s) successive(s),
- déprotéinisation par précipitation ou adsorption sélective,
- purification de la fraction active contenant les polysaccharides de haut poids moléculaire par ultrafiltration, et
- filtration stérilisante.

## Claims

1. Use, for an immediate tensor effect on the skin, of polysaccharides of high molecular weight comprised between 30 and 2 000 kDa, obtained from oat bran and/or fibers and/or seeds and by the implementation of a process consisting in extracting and purifying polysaccharides of high molecular weight from oat bran and/or fibers and/or seeds and in solubilizing and stabilizing these polysaccharides in water, comprising at least the following stages:
- solubilization of oat bran and/or fibers and/or seeds in a basic solution, and
- successive or simultaneous enzymatic hydrolysis(es) of polysaccharides that are contained in the oat bran and/or fibers and/or seeds, and
- deproteinization,
in a cosmetic composition.

2. Use, to provide a stretched and toned skin, of polysaccharides of high molecular weight comprised between 30 and 2 000 kDa, obtained from oat bran and/or fibers and/or seeds and by the implementation of a process consisting in extracting and purifying polysaccharides of high molecular weight from oat bran and/or fibers and/or seeds and in solubilizing and stabilizing these polysaccharides in water, comprising at least the following stages:
- solubilization of oat bran and/or fibers and/or seeds in a basic solution, and
- successive or simultaneous enzymatic hydrolysis(es) of polysaccharides that are contained in the oat bran and/or fibers and/or seeds, and
- deproteinization,
in a cosmetic composition.

3. Use, for an immediate anti-wrinkle effect, of polysaccharides of high molecular weight comprised between 30 and 2 000 kDa, obtained from oat bran and/or fibers and/or seeds and by the implementation of a process consisting in extracting and purifying polysaccharides of high molecular weight from oat bran and/or fibers and/or seeds and in solubilizing and stabilizing these polysaccharides in water, comprising at least the following stages:
solubilisation of oat bran and/or fibers and/or seeds in a basic solution, and
- successive or simultaneous enzymatic hydrolysis(es) of polysaccharides that are contained in the oat bran and/or fibers and/or seeds, and
- deproteinization,
in a cosmetic composition.

4. Use according to any of the preceding claims, wherein said polysaccharides are obtained by the implementation of a process the series of the following stages:
- Solubilization of oat bran and/or fibers and/or seeds in a basic solution, at a rate of 30 g/l to 300 g/l,
- Successive or simultaneous enzymatic hydrolysis(es) of polysaccharides that are contained in the oat bran and/or fibers and/or seeds,
- Inactivation by heat or chemical treatment to block the enzymatic reactions,
- Separation of soluble and insoluble phases by filtration, decanting, and/or centrifuging,
- Successive concentration(s),
- Deproteinization by precipitation or selective adsorption,
- Purification of the active fraction that contains the polysaccharides of high molecular weight by ultrafiltration, and
- Sterilizing filtration.

## Patentansprüche

1. Verwendung von Polysacchariden mit hohem Molekulargewicht zwischen 30 und 2000 kDa zum Erzielen eines sofortigen Hautstraffungseffekts, die ausgehend von Kleie und/oder Fasern und/oder Körnern von Hafer und durch Ausführung eines Verfahrens erhalten werden, das darin besteht, die Polysacchariden mit hohem Molekulargewicht ausgehend von Kleie und/oder Fasern und/oder Körnern von Hafer zu extrahieren und zu reinigen, die Polysacchariden in Wasser zu lösen und zu stabilisieren, wobei das Verfahren wenigstens folgenden Verfahrensschritte aufweist:
- Lösen von Kleie und/oder Fasern und/oder Körnern von Hafer in einer basischen Lösung,
- sukzessive oder gleichzeitige enzymatische Hydrolyse(n) der in der Kleie und/oder den Fasern und/oder den Körnern von Hafer enthaltenen Polysacchariden und
- Deproteinisierung,
in einer kosmetischen Zusammensetzung.

2. Verwendung von Polysacchariden mit hohem Molekulargewicht zwischen 30 und 2000 kDa zur Pflege gespannter und tonischer Haut, die ausgehend von Kleie und/oder Fasern und/oder Körnern von Hafer und durch Ausführung eines Verfahrens erhalten werden, das darin besteht, die Polysacchariden mit hohem Molekulargewicht ausgehend von Kleie und/oder Fasern und/oder Körnern von Hafer zu extrahieren und zu reinigen, die Polysacchariden in Wasser zu lösen und zu stabilisieren, wobei das Verfahren wenigstens folgenden Verfahrensschritte aufweist:
- Lösen von Kleie und/oder Fasern und/oder Körnern von Hafer in einer basischen Lösung,
- sukzessive oder gleichzeitige enzymatische Hydrolyse(n) der in der Kleie und/oder den Fasern und/oder den Körnern von Hafer enthaltenen Polysacchariden und
- Deproteinisierung,
in einer kosmetischen Zusammensetzung.

3. Verwendung von Polysacchariden mit hohem Molekulargewicht zwischen 30 und 2000 kDa zum Erzielen eines sofortigen gegen Falten wirkenden Effekts, die ausgehend von Kleie und/oder Fasern und/oder Körnern von Hafer und durch Ausführung eines Verfahrens erhalten werden, das darin besteht, die Polysacchariden mit hohem Molekulargewicht ausgehend von Kleie und/oder Fasern und/oder Körnern von Hafer zu extrahieren und zu reinigen, die Polysacchariden in Wasser zu lösen und zu stabilisieren, wobei das Verfahren wenigstens folgenden verfahrensschritte aufweist:
- Lösen von Kleie und/oder Fasern und/oder Körnern von Hafer in einer basischen Lösung,
- sukzessive oder gleichzeitige enzymatische Hydrolyse(n) der in der Kleie und/oder den Fasern und/oder den Körnern von Hafer enthaltenen Polysacchariden und
- Deproteinisierung,
in einer kosmetischen Zusammensetzung.

4. Verwendung von Polysacchariden mit hohem Molekulargewicht nach einem der vorhergehenden Ansprüche, **dadurch gekennzeichnet, dass** die Polysacchariden durch Ausführung eines Verfahrens erhaltene Polysaccharide sind, das folgende Verfahrensschritte aufweist:
- Lösen von Kleie und/oder Fasern und/oder Körnern von Hafer in einer basischen Lösung, mit einem Gehalt von 30g/l bis 300g/l,
- sukzessive oder gleichzeitige enzymatische Hydrolyse(n) der in der Kleie und/oder den Fasern und/oder den Körnern von Hafer enthaltenen Polysacchariden,
- Inaktivierung durch thermische oder chemische Behandlung, um enzymatische Reaktionen zu blockieren,
- Trennung der löslichen und unlöslichen Phasen durch Filtration, Abgießen und/oder Zentrifugieren,
- sukzessive Anreicherung(en),
- Deproteinisierung durch Fällung oder selektive Adsorption,
- Reinigung des aktiven Anteils, der Polysaccharide mit hohem Molekulargewicht enthält, durch Ultrafiltration, und
- sterilisierende Filtration.
